# EUROPEAN PATENT SPECIFICATION

(11) **EP 2 131 651 B1**
(45) Date of publication and mention of the grant of the patent: **22.05.2013**
(21) Application number: 08732047.9
(22) Date of filing: 12.03.2008
(51) Int. Cl.: A61K 38/13, A61K 9/00, A61K 38/10, A61K 9/06, A61K 9/08, A61K 9/70, A61K 31/485, A61K 45/06, A61P 29/00, A61P 27/02

(54) **TOPICAL CORNEAL ANALGESIA USING NEUROTENSIN RECEPTOR AGONISTS**
TOPISCHE HORNHAUTSCHMERZMITTEL MIT NEUROTENSIN-REZEPTORAGONISTEN
ANALGÉSIE TOPIQUE DE LA CORNÉE UTILISANT DES AGONISTES DES RÉCEPTEURS DE LA NEUROTENSINE

(30) Priority: 12.03.2007 US 906618 P; 14.05.2007 US 930243 P; 19.07.2007 US 950772 P; 17.12.2007 US 14316 P
(43) Date of publication of application: 16.12.2009
(73) Proprietor: Sarentis Therapeutics, Inc., New York, NY 10021-1811 (US); Sloan-Kettering Institute for Cancer Research, New York, NY 10065 (US); Mayo Foundation for Medical Education and Research, Rochester, MN 55905 (US)
(72) Inventor: BARBUT, Denise, New York, New York 10021 (US); PASTERNAK, Gavril, W., New York, New York 10028 (US); RICHELSON, Elliott, Ponte Vedra, Florida 32082 (US)
(74) Representative: Baldock, Sharon Claire
(86) International application number: PCT/US2008/056721
(87) International publication number: WO 2008/112807

(56) References cited:
- WO-A1-99/52539
- US-A- 4 839 174
- US-A- 5 545 617
- US-A- 5 672 584
- US-A1- 2003 225 360
- US-A1- 2004 018 976
- US-A1- 2005 020 519
- US-A1- 2006 128 610
- US-A1- 2006 189 536
- US-A1- 2006 210 604
- US-A1- 2006 216 317
- US-A1- 2006 247 156
- US-A1- 2007 054 843
- US-B1- 6 294 553
- US-B2- 7 141 547
- FANTEGROSSI W E ET AL: "Antinociceptive, hypothermic, hypotensive, and reinforcing effects of a novel neurotensin receptor agonist, NT69L, in rhesus monkeys", PHARMACOLOGY BIOCHEMISTRY AND BEHAVIOR, ELSEVIER, US, vol. 80, no. 2, 1 February 2005 (2005-02-01), pages 341-349, XP004725318, ISSN: 0091-3057, DOI: 10.1016/J.PBB.2004.12.005

## Description

This is an international filing that claims the benefit of U.S. Application Serial Nos. 60/906,618, filed March 12, 2007; 60/930,243, filed May 14, 2007; 60/950,772, filed July 19, 2007; and 61/014,316, filed December 17, 2007. This is also related to U.S. Application Serial No. 11/709,991, filed February 23, 2007, which claims the benefit of U.S. Application Serial Nos. 60/785,233, filed March 22, 2006 and 60/776,248, filed February 24, 2006.

### BACKGROUND

Nerve endings are abundant in the cornea. It is one of the most densely innervated parts of the body. This is why the eye is very sensitive. This is a protective mechanism that prevents injury to the cornea.

Increasingly, eye surgery is being performed with regional or topical anesthesia. The mainstay for topical anesthesia is the topical anesthetic agent, such as benzocaine or lidocaine. These agents, however, are relatively short acting, with the effect lasting only about 20 minutes. Furthermore, ophthalmologists are reluctant to use these topical anesthetics for post-operative pain because they delay wound healing. Other topical or systemic agents also used in this setting, such as non-steroidal anti-inflammatory drugs (NSAIDs), also delay corneal re-epithelialization. The management of post-operative eye pain is particularly problematic in procedures for correcting refractive errors. In this setting, pain is very significant for about 3-5 days.

There are no known compounds that provide topical analgesia (insensibility to pain without loss of sensation). Ideally, a topical analgesic for the eye would have the following properties: (1) a profound analgesic (loss of pain perception) effect; (2) analgesia would be long lasting (>30 minutes); (3) not be anesthetic (loss of sensation) (because this can make further corneal injury more likely and may be why local anesthetics delay epithelialization); and (4) not delay wound healing or epithelialization.
The following documents disclose that neurotensin analogs are antinociceptive when administered systemically:
WO 99/52539 A1 (MAYO FOUNDATION [US]; RICHELSON ELLIOTT [US]; CUSACK BERNADETTE MARIE) 21 October 1999
FANTEGROSSI W E ET AL: "Antinociceptive, hypothermic, hypotensive, and reinforcing effects of a novel neurotensin receptor agonist, NT69L, in rhesus monkeys", PHARMACOLOGY BIOCHEMISTRY AND BEHAVIOR, ELSEVIER, US, vol. 80, no. 2, 1 February 2005, pages 341-349,

Neurotensins are powerful analgesics. Endogenous neurotensin has to be injected intraventricularly to produce analgesia because it is rapidly digested by peptidases when given systemically. Recently, peptidase resistant variants have been synthesized that can be given intravenously and produce significant analgesia. Many of these compounds, however, are associated with hypotension and may not be able to be given systemically at doses sufficient to produce analgesia.

### SUMMARY OF THE INVENTION

Neurotensins (NTs) and their analogs (or derivatives) provide a combination, if not all of the above requirements for a topical analgesic. The analogs may include neo-tryptophan located at position 11 of the natural neurotensin sequence.

A method for administering an ocular analgesic is described. The method includes the steps of providing a topical analgesic that includes a neo-tryptophan-containing neurotensin analog and applying the topical analgesic to the ocular tissue in a dose of 0.0005 to 1.2 mg, alternatively about 0.0005 to about 1.0 mg, alternatively about 0.00075 to about 1.0 mg, alternatively about 0.001 mg to about 1.0 mg, alternatively about 0.001 mg to about 0.8 mg, alternatively about 0.001 mg to about 0.7 mg, alternatively about 0.001 mg to about 0.6 mg. The neo-tryptophan-containing neurotensin analog may be provided in a concentration of about 0.01 to 12 mg/ml, alternatively about 0.6 to 1.2 mg/ml, alternatively about 0.1 to 10 ml/ml. The amount administered may be approximately 25-75 µl, alternatively approximately 30-60 µl, alternatively approximately 40-55 µl, alternatively approximately 50 µl, or about 1 eyedropful.

The method for administering an ocular analgesic could be performed to treat dry eyes or regular ocular trauma. Alternatively or additionally, the method may include the step of performing LASIK eye surgery, refractive index surgery, cataract surgery, or retinal surgery before the topical analgesic is applied. The topical analgesic could be applied to ocular tissue located near an incision. Alternatively, the topical analgesic could be applied to corneal tissue.

In another embodiment, the invention includes an ocular analgesic for topical administration. The ocular analgesic includes a buffered salt solution and a neo-tryptophan-containing neurotensin analog. The dose of the neo-tryptophan-containing neurotensin analog is 0.0005 to 1.2 mg, alternatively about 0.0005 to about 1.0 mg, alternatively about 0.00075 to about 1.0 mg, alternatively about 0.001 mg to about 1.0 mg, alternatively about 0.001 mg to about 0.8 mg, alternatively about 0.001 mg to about 0.7 mg, alternatively about 0.001 mg to about 0.6 mg. The neo-tryptophan-containing neurotensin analog may be provided in a concentration of about 0.01 to about 2.0 mg/ml, alternatively about 0.1 to about 1.2 mg/ml, alternatively about 0.6 to about 1.2 mg/ml. The ocular analgesic may further include lubricant eye drops, artificial tears, methylcellulose drops, or morphine drops.

In another embodiment, the invention includes an ocular analgesic for topical administration that has a local anesthetic and a neo-tryptophan-containing neurotensin as described above. The local anesthetic may be proparacaine, benzocaine, bupivacaine, lidocaine, mepivacaine, procaine, tetracaine, ropivacaine, proparacaine, etidocaine, prmaoxine, cocaine, or butamben. Alternatively, the local anesthetic may be a sodium channel blocker.

In another embodiment, the invention includes an ocular analgesic for topical administration that has a neo-tryptophan-containing neurotensin analog and a tissue penetrating agent. The neo-tryptophan-containing neurotensin analog may be provided in a concentration of about 0.01 to about 100 mg/ml, alternatively about 0.01 to about 75 mg/ml, alternatively about 0.01 to about 50 mg/ml, alternatively about 0.05 to about 40 mg/ml, alternatively about 0.1 to about 5 mg/ml, alternatively about 0.3 to about 4 mg/ml, alternatively about 0.5 to about 3 mg/ml. Alternatively, the concentration may be from about 1.0 to about 10 mM, alternatively from about 0.1 to about 100 mM, alternatively from about 0.01 to about 100 mM. The dose administered may be applied over the region affected. The tissue penetrating agent may be DMSO, emulsifying wax, gel, methylcellulose, methylparaben, mineral oil, poloxamer 188, propylene glycol, or white petrolatum. The invention may be in the form of a cream or ointment.

In another embodiment, the invention includes a patch for administering a topical analgesic. The patch includes a drug reservoir film containing neurotensin, neurotensin analog, or neurotensin receptor agonist, a transdermal patch film, and a porator film. The neurotensin, neurotensin analog, or neurotensin receptor agonist may be provided in a concententration of about 0.05 to about 40 mg/ml, alternatively about 0.1 to about 5 mg/ml, alternatively about 0.3 to about 4 mg/ml, alternatively about 0.5 to 3 mg/ml. The dose administered may be about 1 to about 10 ml, alternatively about 1 to about 7 ml, alternatively about 1 to about 5 ml. The dose administered may be about 1 mg to about 50 mg, alternatively about 1 mg to about 40 mg, alternatively about 1 mg to about 30 mg, alternatively about 1 mg to about 25 mg, alternatively about 1 mg to about 25 mg. The rate of release/administration may be at least about 0.20 µg/min, alternatively at least about 1.0 µg/min, alternatively at least about 2.0 µg/min, alternatively at least about 10.0 µg/min, alternatively at least about 15.0 µg/min, alternatively at least about 20.0 µg/min, alternatively at least about 50 µg/min, alternatively at least about 100.0 µg/min, alternatively at least about 150.0 µg/min, alternatively at least about 200.0 µg/min, alternatively at least about 1000.0 µg/min, alternatively at least about 2000.0 µg/min. The neurotensin analog may be a neo-tryptophan containing neurotensin analog. The drug reservoir film may be made of EVA polymer, mannitol, and hydromorphone HCl.

The neo-tryptophan-containing neurotensin analogs of the above-described embodiments could be a hexapeptide or pentapeptide analog that contains neo-tryptophan (at position 11 of the natural neurotensin sequence). The neo-tryptophan-containing neurotensin analog may be, but is not limited to, NT71, NT72, NT69, NT67, or NT76.

### BRIEF DESCRIPTION OF THE DRAWINGS

FIGURE 1. Graph of tail flick latency (sec) after the distal portions of mice tails were immersed in a DMSO solution of NT72.

FIGURE 2. Graph of tail flick latency (sec) after the distal portions of mice tails were immersed in a DMSO solution of neurotensin analogs (graded response).

FIGURE 3. Graph of analgesia (percent of mice) after the distal portions of mice tails were immersed in DMSO solutions of neurotensin analogs (quantal response).

FIGURE 4. Graph of topical effect of neurotensin analogs in rabbit intact cornea (Time (min) vs. No. of Blinks).

FIGURES 5A-C. Graph of topical dose response curves for NT71, NT72, and NT69 in rabbit intact cornea (Dose (mg/ml) v. No. of Blinks). A. NT71 and NT72, 15-min post-dose; B. NT71 and NT72, 30-min post-dose, C. NT69, 30-min post-dose.

FIGURE 6. Graph of cell migration assay from *in vitro* scratch assay (Compound vs. Migration (µm)). "PROP,"proparacaine; "TET," tetracaine.

FIGURE 7. A line plot showing percent of scratch closure of corneal epithelial cells *in vitro* (Time (hours) vs. Closure (%)). "PROP,"proparacaine; "TET," tetracaine.

FIGURE 8. A bar graph showing percent of scratch closure of corneal epithelial cells *in vitro* after administration of various compounds (Time (hours) vs. Closure (%)). "PROP,"proparacaine; "TET," tetracaine.

FIGURE 9. A bar graph showing percent of scratch closure of corneal epithelial cells *in vitro* after administration of various compounds (proparacaine, tetracaine, SAR001 (NT71), and SAR002 (NT72)) (Compound vs. Closure (%)).

FIGURES 10A-B. Graph of *in vitro* cytotoxicity results from studies in intact rabbit cornea after addition of 70% MeOH, proparacaine, tetracaine, NT71, or NT72 (Concentration (mM or mg/ml) vs. % Cells Living).

FIGURE 11. Graph of wound healing following superficial keratectomy in *vivo* (Time Following Procedure (hours) vs. Wound Closure (%)).

### DETAILED DESCRIPTION

Neurotensins (including neurotensin, neurotensin analogs, or neurotensin receptor agonists), alone or in combination with opiates, have been found to have significant topical analgesic effect. Various neurotensin receptor agonists include, without limitation, those described in U.S. Patent Nos. 6,858,396; 6,783,946; 6,566,330; 6,358,922; and 6,043,218. The sequences of compounds NT69, NT71, and NT72, which are neurotensin analogs that contain *neo*-Trp at position 11 of the natural neurotensin sequence. , are listed in TABLE 1 below. These neo-tryptophan-containing analogs are resistant to degradation by proteases. Other neo-tryptophan containing neurotensin analogs can be found in U.S. Application Serial Nos. 11/800,975, filed May 7, 2007 and 11/709,991, filed February 23, 2007.

**TABLE 1: Neurotensin Analog Sequences**

| **Polypeptide** | **Position** | | | | | |
|---|---|---|---|---|---|---|
| | **8** | **9** | **10** | **11** | **12** | **13** |
| NT67 (a.k.a. NT67L) | D-Lys | L-Arg | L-Pro | L*-neo-*Trp | L-Ile | L-Leu |
| NT69 (a.k.a. NT69L) | N-methyl-Arg | L-Lys | L-Pro | L*-neo-*Trp | tert-Leu | L-Leu |
| NT71 (a.k.a. SAR001) | N-methyl-Arg | DAB | L-Pro | L*-neo-*Trp | tert-Leu | L-Leu |
| NT72 (a.k.a. SAR002) | | D-Lys | L-Pro | L*-neo-*Trp | tert-Leu | L-Leu |
| NT76 | L-Arg | D-Orn | L-Pro | L*-neo-*Trp | L-Ile | L-Leu |

| | | | | | | |
|---|---|---|---|---|---|---|
| Abbreviations: DAB = diaminobutyric acid; NT=neurotensin; *neo*-Trp = a regio-isomer of the native tryptophan (See Fauq, A.H. et al. "Synthesis of (2S)-2-amino-3-(1H-4-indolyl)propanoic acid, a novel tryptophan analog for structural modification of bioactive peptides." Tetrahedron: Asymmetry 9: 4127-34 (1998)) | | | | | | |

Topical application of neurotensins (NTs) has the advantage of eliminating systemic side-effects attributable to the drug, such as hypotension, which may be problematic with neurotensins that bind both NTS1 1 and NTS2 receptors. Systemic absorption (blood levels) following topical application for any NT compound is known to be minimal because:
1. Typically, blood levels are <10% of total dose following topical administration of drugs in the eye, such as beta-blockers. Hypotension, for example, one of the main effects of beta-blockers, is not associated with topical use.
2. Topical application of neurotensin on rat tail in a DMSO solution caused analgesia in the segment of the tail covered with the drug but not a neighboring segment of the tail not exposed to the drug, indicating that systemic absorption did not occur.

### EXAMPLE 1: Determination of Topical Analgesic Effect of Neurotensin Analogs in a Mouse Tail flick Model

Antinociception (reduction of sensitivity to painful stimuli) can be measured using pain tests such as tail flick studies. Tail flick studies typically involve subjecting an animal to a painful stimulus (e.g., heat or a pin prick), and measuring the length of time or amount of pinching force applied before the animal physically responds to the stimulus by flicking its tail.

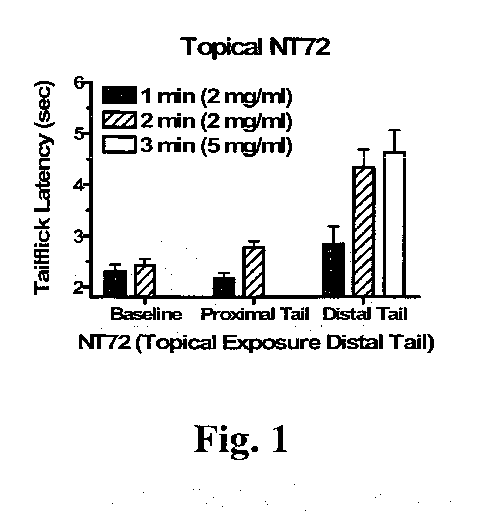

In this mouse tail flick model, the distal end region of the mouse's tail was immersed approximately 3 cm into a DMSO solution containing NT72 at concentrations of 2 mg/ml and 5 mg/ml. Analgesia was then determined using a radiant heat tailflick test in which a focused beam of light was targeted on the tail and latency before the animal removed its tail from the painful stimulus was measured. As seen in **FIGURE 1****,** there was little effect on the proximal tail (*i.e.,* the portion not exposed to the drug) when compared to baseline. The portion of the tail exposed to NT72, however, showed a very robust result with the animals waiting at least 4 seconds before removing their tails. As apparent from the increased latency of tail flicks, NT72 induces analgesia in this portion of the tail but not elsewhere. Therefore, the analgesic effect produced is a local one and not secondary to systemic absorption of the drug. Additionally, the analgesic effect was as robust as that of morphine (not shown).

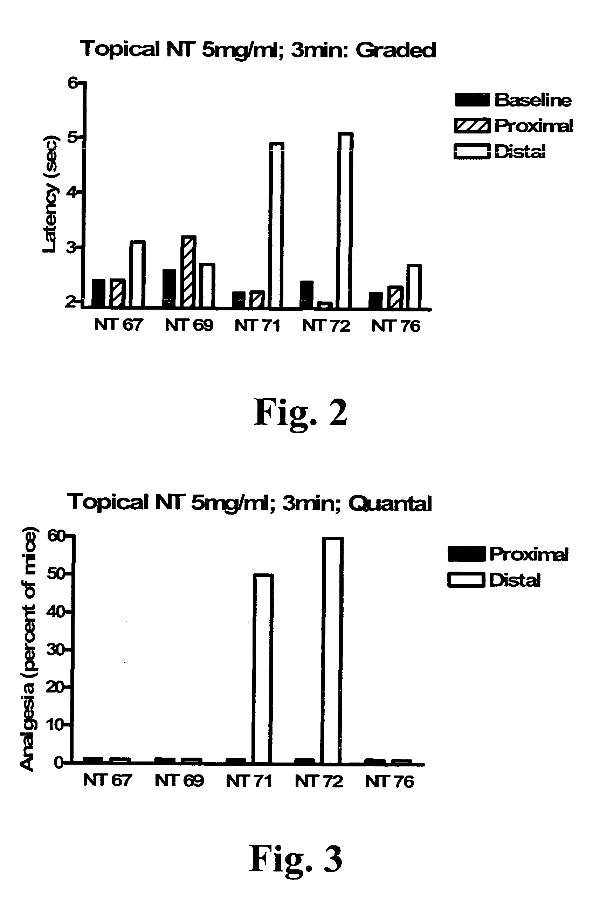

**FIGURES 2** and **3** illustrate the topical analgesic effect of various neurotensin analogs (NT67, NT69, NT71, NT72, and NT76). NT71 and NT72 applied to the distal third of a mouse tail showed a more robust analgesia than the other neurotensin analogs, with the animals waiting approximately 5 seconds before they reacted to the painful stimulus (see FIGURE 2). As seen in FIGURE 3, an analgesic effect (> 30% increase in latency) was found in about 50% of the mice tested for NT71 and in about 60% of the mice tested for NT72.

### EXAMPLE 2: Determination of Topical Corneal Analgesic Effect in an Ocular Pain Model in Rabbit

The purpose of the study was to determine whether neurotensin analogs had topical analgesic action in the rabbit eye. New Zealand white rabbits were used and corneal analgesia was measured using a Cochet-Bonnet Aesthesiometer.

Twenty-four rabbits, each weighing 2-3 kg, were tested. Animals were randomized to three groups. Each group of two animals received one of three neurotensin compounds, NT69, NT71 (SAR001), or NT72 (SAR002). Baseline corneal sensation was determined using a hand-held Cochet-Bonnet aesthesiometer (Luneau Ophthalmologie, Paris, France). To obtain a measurement, the cornea was viewed at close range from the side while the aesthesiometer was advanced with perpendicular corneal contact until flexure of the filament was observed. The lowest pressure was used initially (30-40 mm filament length), and measurements proceeded in an ascending fashion. The highest pressure was at a filament length of 10 mm. At each predetermined length, six measurements were made. From these measurements, the number of blinks (out of 6) that occurred at any given length was calculated at baseline (BSS or saline control), and at 15, 30, 60, 90, 150, 210, and 240 minutes following the administration of the test compound. The dose of each compound was calculated from the known ED50s of the drugs. A dose that was several fold higher than the ED50 was given, on the assumption that the vast majority would be washed out in tears. Approximately 50-70 µl aliquots of the following solutions were placed in the eyes (NT69 (1.2 mg/ml), NT71 (1 mg/ml), and NT72 (1 mg/ml).

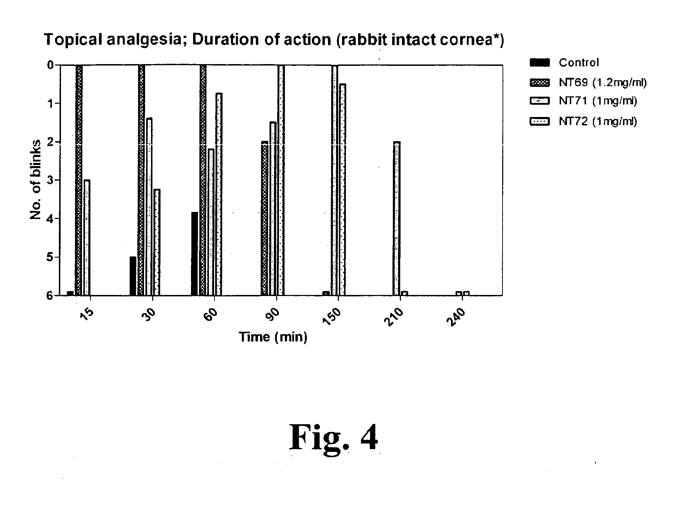

As seen in **FIGURE 4****,** all three neurotensin analogs (NT69, NT71 (SAR001), or NT72 (SAR002)) abolished corneal sensation (no blink response to the strongest stimulus, 10 mm hairs), and for prolonged periods of time. NT69 had a very fast onset (no blink response at 15 minutes after administration) that lasted for at least 90 minutes. NT71 and NT72 had slower onsets compared to NT69 but a longer duration of action - there was no blink response at 30 minutes after administration and the analgesic effect lasted at least 150 minutes for NT72 and at least 210 minutes for NT71.

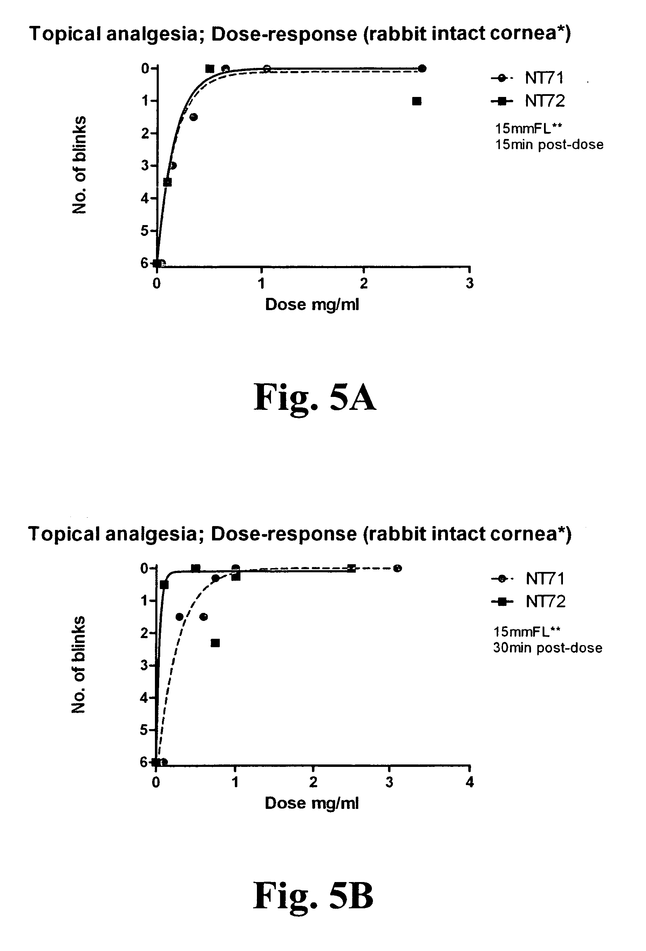

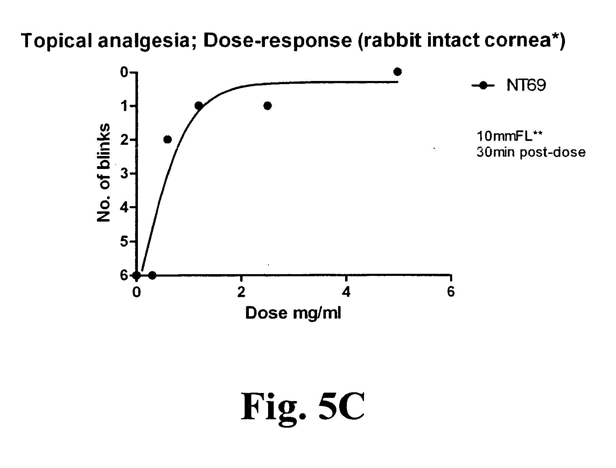

**FIGURES 5A-C** illustrate the dose response curves for NT71 and NT72 at 15 and 30 minutes and NT69 at 30 minutes following topical administration of specific doses of neurotensin analog into the eye. Twenty-four rabbits were each tested three times over a 24-hour period. Baseline was established in both eyes. Approximately 50-70 µl of the indicated concentration of drug was applied to the rabbit's eye. Changes in the blink response were recorded for 4 hours (or until return to baseline). Doses show that the neurotensin analogs induce analgesia in the rabbit eye at doses as low as about 0.1 to about 0.3 mg/ml.

Irritant effects of the compounds and of the saline solution were tested by examining each eye at baseline, after instilling BSS (buffered saline solution), and 5 minutes and 1 hour after administration of test compound. Redness, chemosis, tearing, and corneal opacity were evaluated in all eyes using the Draize scoring method. No abnormalities were noticed.

### EXAMPLE 3. Corneal Wound Healing

### Measurement of Rate of Closure of Wounds in Rabbit Corneal Epithelial Cells

A major drawback of current topical anesthetics is the inhibition of corneal epithelial cell migration with delay of wound healing. Neurotensin compounds deliver analgesic effect without inhibition or delay of corneal healing. This distinguishes the neurotensin topical analgesics from the local anesthetics and from NSAI (non-steroidal anti-inflammatory) eye drops, all of which delay wound healing. This is a very important distinction for the neurotensins because delayed healing increases the risk of complications following eye surgery.

Based on tests of neurotensin analogs in the eye, we have unexpectedly discovered that neurotensins are effective analgesic agents in the eye. Experimental data for NT69, SAR001 (also known as NT71), and SAR002 (also known as NT72) show prolonged and profound analgesia in an established ocular pain model in the rabbit.

### In Vitro Scratch Assay

The limbus was removed from both eyes of anesthetized rabbits and dissected to include 1.0 to 1.5 mm peripheral cornea and 1.0 to 1.5 mm beyond the anatomical limbus. Each limbus was divided into four small segments. Two segments were used for each 60 mm square plate.

Cultured rabbit corneal epithelial cells were utilized to measure the closure of a scratch made in a confluent epithelial monolayer in the presence of varying doses of the test compounds (0.1 mg/ml to 10 mg/ml). Uniform scratches were made in confluent monolayers of primary rabbit corneal epithelial cells *("in vitro* scratch assay").

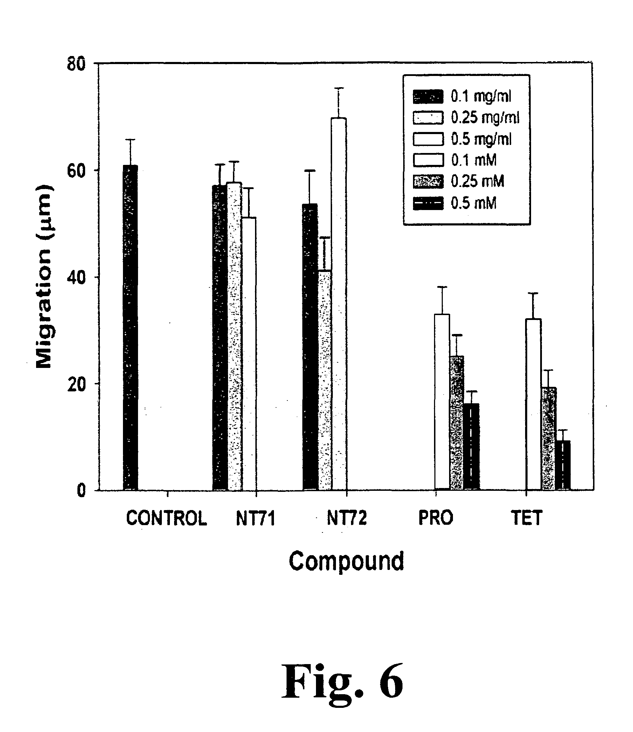

Individual cell migration assays were performed in the presence of the test compounds and compared with standard doses of tetracaine and proparacaine. Movement of cells from the edge of the scratch (wound) toward the middle of the scratch (wound) were measured over 24 hours. As seen in **FIGURE 6****,** cells in which the control (PBS), NT71, or NT72 were added in various concentrations resulted in a much larger migration of cells over the 24 hour period as compared to wounds in which proparacaine and tetracaine were added. Cells in which NT71 and NT72 were added migrated at least 40 µm, alternatively at least 50 µm, alternatively at least 60 µm, alternatively at least 70 µm over 24 hours.

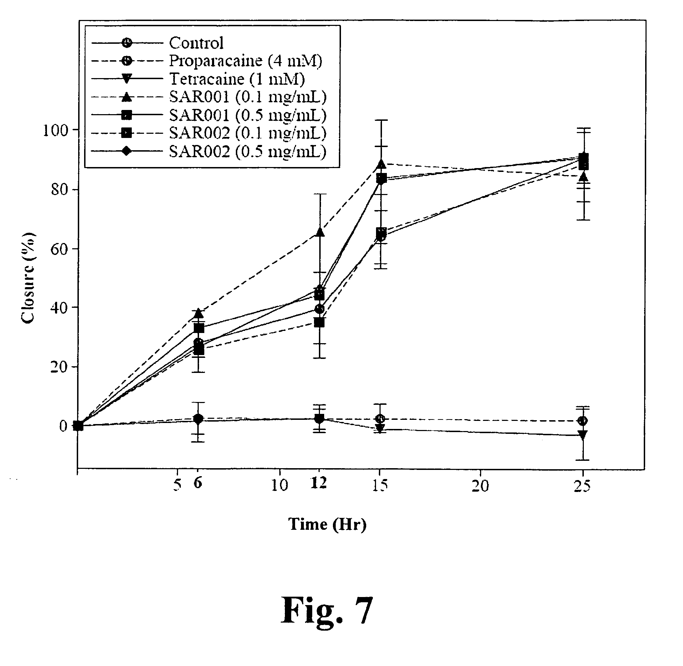

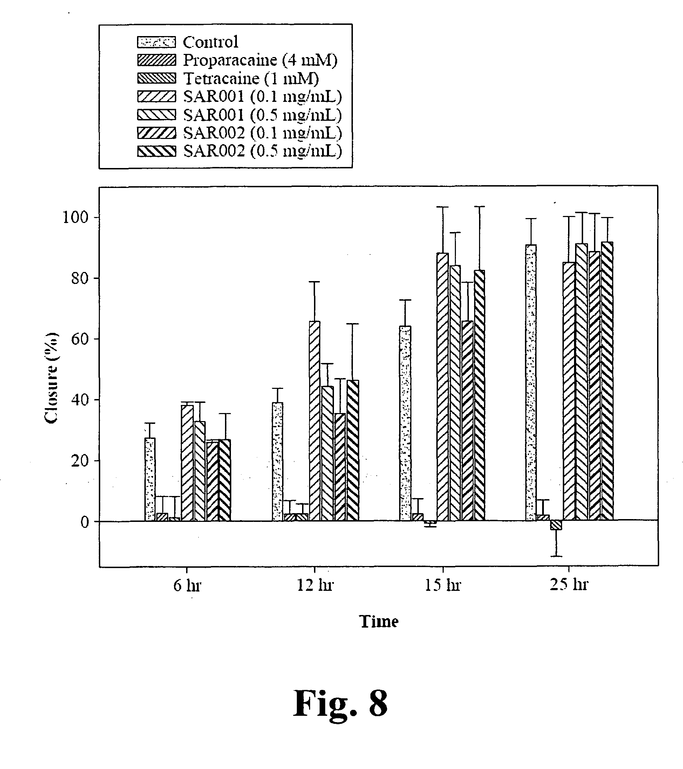

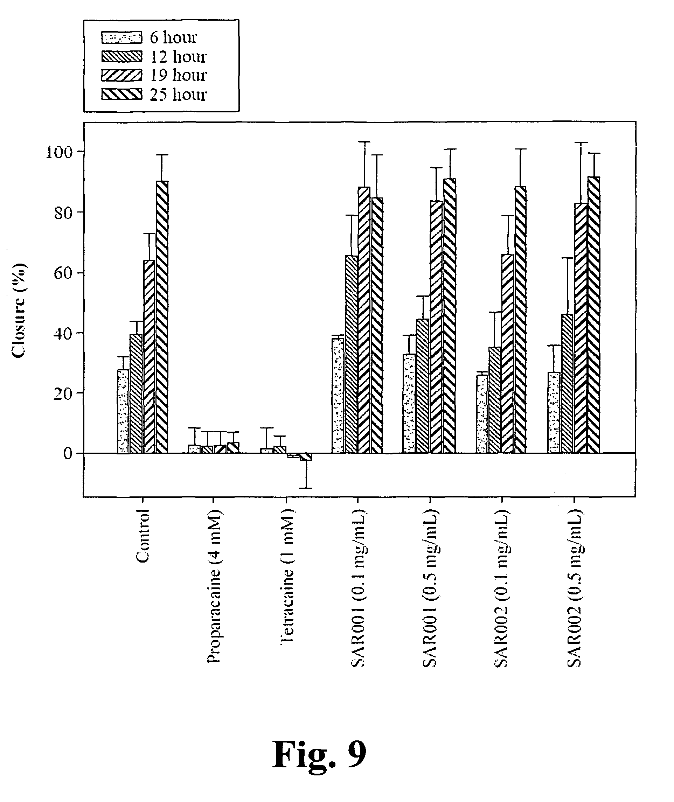

The rate of closure of the *in vitro* wounds were measured in the presence of traditional Na⁺-channel blocking anesthetics (proparacaine or tetracaine) or neurotensin agonists (NT71 (SAR001) or NT72 (SAR002)) at two concentrations (0.1 mg/ml and 0.5 mg/ml). As apparent from **FIGURES 7 - 9****,** the monolayer scratch was closed much more rapidly with neurotensin analogs than with tetracaine or proparacaine. There was very little closure of the wounds in which proparacaine and tetracaine were added. Tetracaine caused the greatest delay in monolayer closure. In fact, scratches treated with either proparacaine or tetracaine did not close. In contrast, control cells substantially closed the defect within 24 hours. SAR001 (NT71) and SAR002 (NT72) at both lower (0.1 mg/ml) and higher (0.5 mg/ml) doses did not inhibit the rate of scratch closure as compared to control. In fact, low dose SAR001 (NT71) and high dose SAR002 (NT72) may accelerate rate of scratch closure. Scratches in which neurotensin analogs (SAR001 (NT71) and SAR002 (NT72)) were added showed at least 80% closure within at least 15 hours, alternatively within at least 20 hours, alternatively within at least 25 hours. Additionally, many concentrations of the neurotensin analogs resulted in faster healing over the control (PBS).

### In Vitro Cytotoxicity Studies

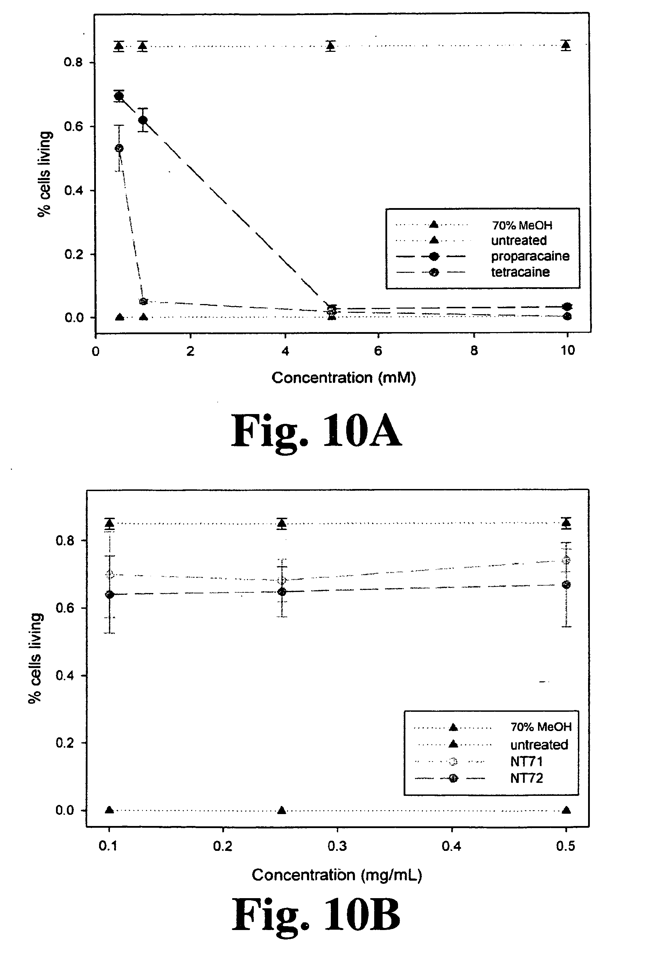

*In vitro* cytotoxicity studies were performed in intact rabbit cornea. Proparacaine (0.5 mM, 1 mM, 5 mM, and 10 mM), tetracaine (0.5 mM, 1 mM, 5 mM, and 10 mM), NT71 (0.1 mg/ml (0.13 mM), 0.25 mg/ml (0.32 mM), and 0.5 mg/ml (0.63 mM)), NT72 (0.1 mg/ml (0.15 mM), 0.25 mg/ml (0.38 mM), and 0.5 mg/ml (0.76 mM)), and 70% MeOH were added to intact rabbit corneas. The percentage of living cells was determined over a 24 hour period using light microscopy and fluorescence. As apparent from FIGURE 10A, tetracaine was cytotoxic to the corneal cells at concentrations as low as 0.5 mM, resulting in the death of about half of the corneal cells observed. Administration of 1 mM tetracaine resulted in the death of almost all of the corneal cells observed. Similarly, administration of 5 mM proparacaine resulted in the death of almost all of the corneal cells. In contrast, as seen in **FIGURE 10B****,** administration of NT71 and NT72 resulted in very limited cell toxicity (less than approximately 20 % of the corneal cells died, alternatively less than approximately 25% of the corneal cells died, alternatively less than approximately 30% of the corneal cells died, compared to untreated cells).

### In Vivo Superficial Keratotomy

### Administration of Neurotensin Analogs Every 2 Hours for 36 Hours

*In vivo* superficial keratotomy experiments were performed on rabbits to access healing effects in an animal model.

Starting with equivalent circular wounds of 6.5 mm diameter (33 mm²), after 36 hrs of administration every 2 hours of 1 mg/mL NT71(SAR001), NT72 (SAR002), tetracaine or saline (control), the persistent epithelial defect (wound) size was:
Control (saline) 4 mm²
Tetracaine 14.4 mm²
SAR001 2.8 mm²
SAR002 4.8 mm²

After 48 hours, wounds treated with control (saline), NT71 (SAR001), and NT72 (SAR002) were fully healed. The tetracaine residual epithelial defect was still present.

Administration every 2 hours of neurotensins at 1 mg/mL in a cornea with open epithelium did not cause any notable adverse ocular effect in the rabbits slit lamp examination. No additional conjunctival injection, no corneal opacification, no anterior chamber cell or flare as compared to control.

Thus, unlike proparacaine and tetracaine, which inhibit epithelial cell proliferation and therefore inhibit healing, SAR001 (NT71) and SAR002 (NT72) can be used in pain management without a compromise in healing. For example, 0.1 mg/ml or less, 0.5 mg/ml or less, 1 mg/ml or less, 3 mg/ml or less, 5 mg/ml or less, 7 mg/ml or less, or 12 mg/ml or less of neurotensin or neurotensin analog can be applied as a topical analgesic for pain management following eye surgery and the patient will experience substantial healing in about the same time as if no analgesic had been used for pain management with use of topical neurotensin analgesics. Substantial healing may occur in 90% or less of the time as if no analgesics had been applied, alternatively 100% or less, alternatively 110% or less, alternatively 120% or less, alternatively 150% or less, or alternatively 200% or less. Substantial healing may occur within 24 hours or less, alternatively 36 hours or less, alternatively 48 hours or less, alternatively 60 hours or less, alternatively 72 hours or less, or alternatively 84 hours or less.

### Administration of Neurotensin Analogs Every 4 Hours for 5 Days

Anesthetized rabbits were placed under an operating microscope, a sterile lid speculum placed in the right eye, and a 6.5 mm trephine used to make a partial thickness epithelial cut centered on the pupil. A sterile beaver blade was used to remove the epithelium within the area of demarcation. A single drop of moxifloxacin 0.1% was instilled at the end of the procedure. The speculum was removed and the animal allowed to recover from the systemic anesthesia. The topical agent under investigation was placed in the eye every 4 hours for 5 days. Neurotensin analogs SAR001 (NT71), SAR002 (NT72), and 69 (NT 69) at 0.6 mg/ml or 0.5% proparacaine or vehicle were instilled at the end of the procedure. The control animals underwent the same surgical procedure. At the end of the procedure, 0.5% proparacaine or vehicle was instilled in the eye every 4 hours.

Fluorescein (1%) was instilled into the eye at times 0, 1, 2, 6, 12, 36, 48, 72, 96, and 120 hours after the procedure and the size of the epithelial defect measured via slit lamp photography. The animals were scored for degree of blinking, tearing, conjunctival injection, anterior chamber inflammation, and intraocular pressure.

The epithelial defect disappeared within 72 hours in all neurotensin treated animals. Disappearance of the defect was delayed to 96-120 hours in the proparacaine-treated animals and was incomplete in 40% of animals at this time point.

### Analgesia and Wound Healing After Excimer Laser Keratectomy

Anesthetized rabbits were placed under an operating microscope, a sterile lid speculum placed in the right eye, and a 6.5 mm excimer laser ablation of 75 microns centered on the pupil was made using a Ladarvision 4000 excimer laser. A single drop of moxifloxacin 0.1 % was used at the end of the procedure. SAR001 (NT71, 0.1% w/w), SAR002 (NT72, 0.1 % w/w), or NT69 was instilled at the end of the procedure and repeated at 4 hour intervals. Control animals underwent the same surgical procedure. Standard doses of tetracaine (0.5% w/w) or proparacaine (not shown) were instilled at the end of the procedure and at 4 hour intervals thereafter.

Fluorescein (1%) was instilled into the right eye at times 0, 1, 2, 6, 12, 36, 48, 72, 96, and 120 hours after the procedure and the size of the epithelial defect measured via slit lamp photography. Degree of blinking, tearing, conjunctival injection, anterior chamber inflammation, and intraocular pressure were evaluated.

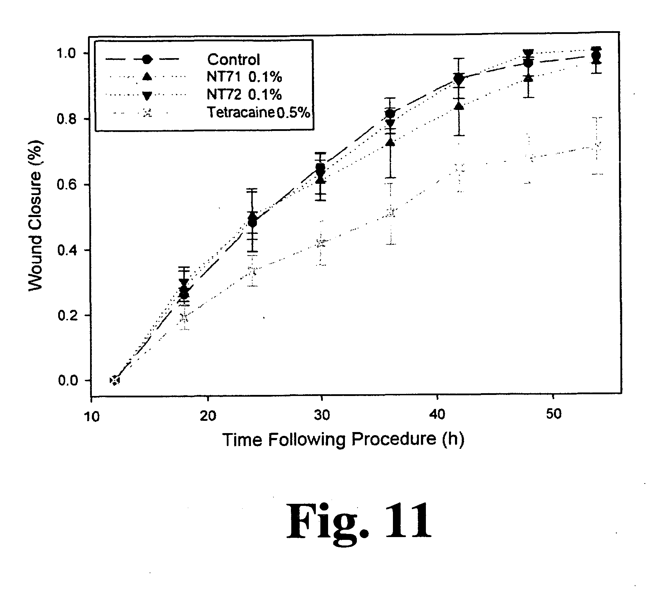

As seen in **FIGURE 11****,** the epithelial defect disappeared within 48 hours in the control and neurotensin analog-treated animals. Furthermore, the wound was closed at least 80% in at least about 36 hours, alternatively at least 90% in at least about 40 hours, alternatively at least about 100% in at least about 48 hours in the neurotensin analog-treated animals. Disappearance of the defect was delayed 96-110 hours in the tetracaine-treated animals and was incomplete in 50% of the tetracaine-treated animals.

### Formulations

Neurotensin analogs can be formulated in combination with lubricant eye drops, artificial tears, or methylcellulose drops for the treatment of dry eye syndrome, corneal lacerations, or eye trauma.

These conditions are characterized by pain, which can be relieved by artificial tears and lubricants and by analgesic eyedrops such as neurotensin analogs. Formulations also include combinations of neurotensin analog drops with morphine drips, which combinations are synergistic. Suitable formulations also include neurotensin analogs and local anesthetic eye drops, both of which relieve pain. Local anesthetics such as tetracaine, however, delay wound healing whereas neurotensin analogs do not delay wound healing. In fact, neurotensin analogs were found to accelerate wound healing, thus reducing unwanted side effects of local anesthetics. Combinations of neurotensin analogs formulated with non-steroidal anti-inflammatory eye drop (NSAI ED), and indeed any other eyedrop can be used for relief of eye pain.

Although the foregoing invention has, for the purposes of clarity and understanding, been described in some detail by way of illustration and example, it will be obvious that certain changes and modifications may be practiced.

## Claims

1. The use of a topical analgesic comprising a neo-tryptophan-containing neurotensin analog, wherein the neo-tryptophan-containing neurotensin analog is present in a dose of 0.0005 mg to 1.2 mg in the manufacture of a medicament for the treatment or prevention of ocular pain, wherein the medicament is for application to ocular tissue.

2. The use of claim 1, wherein the neo-tryptophan-containing neurotensin analog is present in a dose of 0.0005 to 1.0 mg.

3. The use of claim 1, wherein the neo-tryptophan-containing neurotensin analog is present in a dose of 0.001 to 1.0 mg.

4. The use of claim 1, wherein the ocular tissue is located near an incision.

5. The use of claim 1, wherein the ocular tissue comprises corneal tissue.

6. The use of claim 1, further comprising the step of performing LASIK eye surgery, PRK surgery, refractive surgery, cataract surgery, or retinal surgery.

7. The use of claim 1, wherein the method is for treatment of dry eyes or regular ocular trauma.

8. The use of claim 1, wherein the neo-tryptophan-containing neurotensin analog is SAR001 (N-methyl-Arg - DAB - L-Pro - *L-neo-Trp - tert*-Leu - L-Leu).

9. The use of claim 1, wherein the neo-tryptophan-containing neurotensin analog is SAR002 (D-Lys - L-Pro - L-*neo*-Trp *- tert*-Leu - L-Leu).

10. The use of claim 1, wherein the neo-tryptophan-containing neurotensin analog is NT69L (N-methyl-Arg - L-Lys - L-Pro - L-*neo*-Trp *- tert*-Leu *-* L-Leu).

11. The use of claim 1, wherein the topical analgesic further comprises a buffered salt solution.

12. The use of claim 1, wherein the topical analgesic further comprises a gel.

13. The use of claim 1, wherein the step of applying the analgesic to ocular tissue does not delay wound healing or corneal epithelialization.

14. An ocular analgesic for topical administration, comprising:
a buffered salt solution; and
a neo-tryptophan-containing neurotensin analog in a dose of 0.0005 to 1.2 mg.

15. The ocular analgesic of claim 14, wherein the neo-tryptophan-containing neurotensin analog is present in a dose of 0.0005 to 1.0 mg.

16. The ocular analgesic of claim 14, wherein the neo-tryptophan-containing neurotensin analog is present in a dose of 0.001 mg to 1.0 mg.

17. The ocular analgesic of claim 14, wherein the neo-tryptophan-containing neurotensin analog is SAR001 (N-methyl-Arg - DAB - L-Pro - L-*neo*-Trp - *tert*-Leu *-* L-Leu).

18. The ocular analgesic of claim 14, wherein the neo-tryptophan-containing neurotensin analog is SAR002 (D-Lys - L-Pro - L-*neo*-Trp *- tert*-Leu - L-Leu).

19. The ocular analgesic of claim 14, wherein the neo-tryptophan-containing neurotensin analog is NT69L (N-methyl-Arg - L-Lys - L-Pro - L-*neo*-Trp - *tert*-Leu *-* L-Leu).

20. The ocular analgesic of claim 14, wherein the neo-tryptophan-containing neurotensin analog is selected from the group consisting of:
NT64L [L-neo-Trp11]NT(8-13),
SAR002D [D-Lys9,D-neo-Trp11,tert-Leu12]NT(9-13),
NT64D [D-neo-Trp11]NT(8-13),
NT73L [D-Lys9,L-neo-Trp11]NT(9-13).
NT65L [L-neo-Trp11, tert-Leu12]NT(8-13),
NT73D [D-Lys9,D-neo-Tn11]NT(9-13),
NT65D [D-neo-Trp11, tert-Leu12]NT(8-13),
NT74L [DAB9,L-neo-Trp11,tert-Leu12]NT(9- 13),
NT66L [D-Lys8, L- neo-Trp11, tert-Leu12]NT(8-13),
NT74D [DAB9,Pro,D-neo-Trp11,tert-Leu12]NT(9-13),
NT66D [D-Lys8, D-neo-Trp11, tert-Leu12]NT(8-13),
NT75L [DAB8,L-neo-Trp11]NT(8-13),
NT67L [D-Lys8, L-neo-Trp11]NT(8-13),
NT75D [DAB8,D-neo-Trp11]NT(8-13),
NT67D [D-Lys8, D-neo-Trp11]NT(8-13),
NT76L [D-Orn9,L-neo-Trp11]NT(8-13),
NT69L [N-methyl-Arg8,L-Lys9,L-neo-Trp11,tert-Leu12]NT(8-13),
NT76D [D-Orn9,D-neo-Trp11]NT(8-13),
NT69D [N-methyl-Arg8, L-Lys9,D-neo-Trp11,tert-Leu12]NT(8-13),
NT77L [D-Orn9,L-neo-Trp11,tert-Leu12]NT(8-13),
SAR001L [N- methyl- Arg8,DAB9,L-neo-Trp11,tert-Leu12]NT(8-13),
NT77D [D-Orn9,D-neo-Trp11,tert-Leu12]NT(8-13),
SAR001D [N-methyl-Arg8,DAB9,D-neo-Trp11,tert-leu12]NT(8-13),
NT78L [N-methyl-Arg8,D-Orn9,L-neo-Trp11,tert-Leu12]NT(8-13),
SAR002L [D-Lys9,L-neo-Trp11,tert-Leu12]NT(9-13), and
NT78D [N-methyl-Arg8,D-Orn9,D-neo-Trp11,tert-Leu12]NT(8-13).

21. The ocular analgesic of claim 14, further comprising an opiate.

22. The ocular analgesic of claim 21, wherein the opiate is selected from the group consisting of morphine, codeine, hydromorphone, oxymorphone, oxydodone, hydrocodone, meperidine, levorphanol, methadone, sufentanil, alfentanil, fentanyl, remifentanil, levomethadyl, nalbuphine, pentazocine, butorphanol, and buprenolphine.

23. The ocular analgesic of claim 14, further comprising a substance from the group consisting of lubricant eye drops, artificial tears, methylcellulose drops, and morphine drops.

24. The ocular analgesic of claim 14, further comprising a substance from the group consisting of NSAI, steroid, beta blocker, anti viral, immunosuppressant, and cyclosporine.

25. The ocular analgesic of claim 14, wherein the ocular analgesic is in the form of an eyedrop.

## Patentansprüche

1. Verwendung eines topischen Schmerzmittels, mit einem Neo-Tryptophan aufweisenden Neurotensin-Analogon, wobei das Neo-Tryptophan aufweisende Neurotensin-Analogon in einer Dosis von 0,0005 mg bis 1,2 mg in der Herstellung eines Medikaments zur Behandlung oder Vorbeugung von Augenschmerzen vorhanden ist, wobei das Medikament zur Auftragung auf Augengewebe ausgestaltet ist.

2. Verwendung nach Anspruch 1, wobei das Neo-Tryptophan aufweisende Neurotensin-Analogon in einer Dosis von 0,0005 mg bis 1,0 mg vorhanden ist.

3. Verwendung nach Anspruch 1, wobei das Neo-Tryptophan aufweisende Neurotensin-Analogon in einer Dosis von 0,001 mg bis 1,0 mg vorhanden ist.

4. Verwendung nach Anspruch 1, wobei das Augengewebe nahe einer Inzision positioniert ist.

5. Verwendung nach Anspruch 1, wobei das Augengewebe Hornhautgewebe aufweist.

6. Verwendung nach Anspruch 1, die des Weiteren den Schritt des Durchführens einer LASIK-Augenoperation, PRK-Operation, refraktiven Chirurgie, Kataraktoperation oder Netzhautoperation aufweist.

7. Verwendung nach Anspruch 1, wobei das Verfahren zur Behandlung von trockenen Augen oder regulärem Augentrauma ausgestaltet ist.

8. Verwendung nach Anspruch 1, wobei das Neo-Tryptophan aufweisende Neurotensin-Analogon SAR001 (N-methyl-Arg - DAB - L-Pro - L-neo-Trp - *tert*-Leu *-* L-Leu) ist.

9. Verwendung nach Anspruch 1, wobei das Neo-Tryptophan aufweisende Neurotensin-Analogon SAR002 (D-Lys - L-Pro - L-neo-Trp - *tert*-Leu *-* L-Leu) ist.

10. Verwendung nach Anspruch 1, wobei das Neo-Tryptophan aufweisende Neurotensin-Analogon NT69L (N-methyl-Arg - L-Lys - L-Pro - L*-neo-*Trp *- tert*-Leu *-* L-Leu) ist.

11. Verwendung nach Anspruch 1, wobei das topische Schmerzmittel des Weiteren eine gepufferte Salzlösung aufweist.

12. Verwendung nach Anspruch 1, wobei das topische Schmerzmittel des Weiteren ein Gel aufweist.

13. Verwendung nach Anspruch 1, wobei der Schritt des Auftragens des Schmerzmittels auf Augengewebe nicht Wundheilung oder korneale Epithelialisierung verzögert.

14. Augenschmerzmittel für topische Verabreichung, das folgendes aufweist:
eine gepufferte Salzlösung; und
ein Neo-Tryptophan aufweisendes Neurotensin-Analogon in einer Dosis von 0,0005 bis 1,2 mg.

15. Augenschmerzmittel nach Anspruch 14, wobei das Neo-Tryptophan aufweisende Neurotensin-Analogon in einer Dosis von 0,0005 bis 1,0 mg vorhanden ist.

16. Augenschmerzmittel nach Anspruch 14, wobei das Neo-Tryptophan aufweisende Neurotensin-Analogon in einer Dosis von 0,001 mg bis 1,0 mg vorhanden ist.

17. Augenschmerzmittel nach Anspruch 14, wobei das Neo-Tryptophan aufweisende Neurotensin-Analogon SAR001 (N-methyl-Arg - DAB - L-Pro - L*-neo-*Trp *- tert*-Leu *-* L-Leu) ist.

18. Augenschmerzmittel nach Anspruch 14, wobei das Neo-Tryptophan aufweisende Neurotensin-Analogon SAR002 (D-Lys - L-Pro - L-neo-Trp - *tert*-Leu *-* L-Leu) ist.

19. Augenschmerzmittel nach Anspruch 14, wobei das Neo-Tryptophan aufweisende Neurotensin-Analogon NT69L (N-methyl-Arg - L-Lys - L-Pro - L-neo-Trp - *tert*-Leu *-* L-Leu) ist.

20. Augenschmerzmittel nach Anspruch 14, wobei das Neo-Tryptophan aufweisende Neurotensin-Analogon aus der Gruppe ausgewählt ist, die aus folgendem besteht:
NT64L [L-neo-Trp11]NT(8-13).
SAR002D [D-Lys9,D-neo-Trp11,tert-Leu12]NT(9-13).
NT64D [D-neo-Trp11]NT(8-13),
NT73L [D-Lys9,L-neo-Trp11]NT-(9-13),
NT65L [L-neo-Trp11, tert-Leu12]NT(8-13),
NT73D [D-Lys9,D-neo-Trp11]NT(9-13),
NT65D [D-neo-Trp11, tert-Leu12]NT(8-13),
NT74L [DAB9,L-neo-Trp11,tert-Leu12]NT(9-13),
NT66L [D-Lys8, L-neo-Trp11, tert-Leu12]NT(8-13),
NT74D [DAB9,Pro,D-neo-Trp11,tert-Leu12]NT(9-13),
NT66D [D-Lys8, D-neo-Trp11, tert-Leu12]NT(8-13),
NT75L [DAB8,L-neo-Trp11]NT(8-13),
NT67L [D-Lys8, L-neo-Trp11]NT(8-13),
NT75D [DABB,D-neo-Trp11]NT(8-13),
NT67D [D-Lys8, D-neo-Trp11]NT(8-13),
NT76L [D-Orn9,L-neo-Trp11]NT(8-13),
NT69L [N-methyl-Arg8,L-Lys9,L-neo-Trp11,tert-Leu12]NT(8-13)_{;}
NT76D [D-Orn9,D-neo-Trp11]NT(8-13).
NT69D [N-methyl-Arg8, L-Lys9,D-neo-Trp11,tert-Leu12]NT(8-13),
NT77L [D-Orn9L-neo-Trp11,tert-Leu12]NT(8-13),
SAR001L [N- methyl- Arg8,DAB9,L-neo-Trp11-tert-Leu12]NT(8-13),
NT77D [D-Orn9,D-neo-Trp11,tert-Leu2]NT(8-3),
SAR001D [N-methyl- Arg8,DAB9,D-neo-Trp11,tert-Leu12]NT(8-13),
NT78L [N-methyl-Arg8,D-Orn9,L-neo-Trp11,tert-Leu12]NT(8-13),
SAR002L [D-Lys9,L-neo-Trp11,tert-Leu12]NT(9-13) und
NT78D [N-methyl-Arg8,D-Orn9,D-neo-Trp11,tert-Leu12]NT(8-13).

21. Augenschmerzmittel nach Anspruch 14, das des Weiteren ein Opiat aufweist.

22. Augenschmerzmittel nach Anspruch 21, wobei das Opiat aus der Gruppe bestehend aus Morphin, Codein, Hydromorphon, Oxymorphon, Oxydodon, Hydrocodon, Meperidin, Levorphanol, Methadon, Sufentanil, Alfentanil, Fentanyl, Remifentanil, Levomethadyl, Nalbuphin, Pentazocin, Butorphanol und Buprenorphin ausgewählt wird.

23. Augenschmerzmittel nach Anspruch 14, das des Weiteren eine Substanz aus der Gruppe bestehend aus benetzenden Augentropfen, Tränenersatzmitteln, Methylcellulose-Tropfen und morphinen Tropfen aufweist.

24. Augenschmerzmittel nach Anspruch 14, das des Weiteren eine Substanz aus der Gruppe bestehend aus NSAI, Steroid, Beta-Blocker, antiviral, Immunosuppressivum und Cyclosporin aufweist.

25. Augenschmerzmittel nach Anspruch 14, wobei das Augenschmerzmittel die Form eines Augentropfens aufweist.

## Revendications

1. Utilisation d'un analgésique topique comprenant un analogue de la neurotensine contenant du néo-tryptophane, dans laquelle l'analogue de la neurotensine contenant du néo-tryptophane est présent en une dose de 0,0005 mg à 1,2 mg dans la fabrication d'un médicament pour le traitement ou la prévention d'une douleur oculaire, dans laquelle le médicament est destiné à l'application sur un tissu oculaire.

2. Utilisation selon la revendication 1, dans laquelle l'analogue de la neurotensine contenant du néo-tryptophane est présent en une dose de 0,0005 à 1,0 mg.

3. Utilisation selon la revendication 1, dans laquelle l'analogue de la neurotensine contenant du néo-tryptophane est présent en une dose de 0,001 à 1,0 mg.

4. Utilisation selon la revendication 1, dans laquelle le tissu oculaire est situé près d'une incision.

5. Utilisation selon la revendication 1, dans laquelle le tissu oculaire comprend le tissu cornéen.

6. Utilisation selon la revendication 1, comprenant en outre l'étape consistant à réaliser une chirurgie oculaire LASIK, une opération PRK, une chirurgie réfractive, une chirurgie de la cataracte ou une chirurgie de la rétine.

7. Utilisation selon la revendication 1, dans laquelle le procédé est destiné au traitement de la sécheresse oculaire ou du traumatisme oculaire classique.

8. Utilisation selon la revendication 1, dans laquelle l'analogue de la neurotensine contenant du néo-tryptophane est le SAR001 (N-méthyl-Arg - DAB -L-Pro -L-néo-Trp - *tert-*Leu - L-Leu).

9. Utilisation selon la revendication 1, dans laquelle l'analogue de la neurotensine contenant du néo-tryptophane est le SAR002 (D-Lys - L-Pro - L-*néo*-Trp - tert-Leu - L-Leu).

10. Utilisation selon la revendication 1, dans laquelle l'analogue de la neurotensine contenant du néo-tryptophane est le NT69L (N-méthyl-Arg - L-Lys - L-Pro -L-*néo*-Trp - tert-Leu - L-Leu).

11. Utilisation selon la revendication 1, dans laquelle l'analgésique topique comprend en outre une solution saline tamponnée.

12. Utilisation selon la revendication 1, dans laquelle l'analgésique topique comprend en outre un gel.

13. Utilisation selon la revendication 1, dans laquelle l'étape consistant à appliquer l'analgésique sur le tissu oculaire ne retarde pas la guérison des plaies ou l'épithélisation de la cornée.

14. Analgésique oculaire pour administration topique, comprenant :
une solution saline tamponnée ; et
un analogue de la neurotensine contenant du néo-tryptophane en une dose de 0,0005 à 1,2 mg.

15. Analgésique oculaire selon la revendication 14, dans lequel l'analogue de la neurotensine contenant du néo-tryptophane est présent en une dose de 0,0005 à 1,0 mg.

16. Analgésique oculaire selon la revendication 14, dans lequel l'analogue de la neurotensine contenant du néo-tryptophane est présent en une dose de 0,001 mg à 1,0 mg.

17. Analgésique oculaire selon la revendication 14, dans lequel l'analogue de la neurotensine contenant du néo-tryptophane est le SAR001 (N-méthyl-Arg - DAB -L-Pro - L-*néo*-Trp - *tert-*Leu *-* L-Leu).

18. Analgésique oculaire selon la revendication 14, dans lequel l'analogue de la neurotensine contenant du néo-tryptophane est le SAR002 (D-Lys - L-Pro - L-*néo*-Trp - *tert*-Leu *-* L-Leu).

19. Analgésique oculaire selon la revendication 14, dans lequel l'analogue de la neurotensine contenant du néo-tryptophane est le NT69L (N-méthyl-Arg - L-Lys - L-Pro - L-*néo*-Trp - *tert-*Leu *-* L-Leu).

20. Analgésique oculaire selon la revendication 14, dans lequel l'analogue de la neurotensine contenant du néo-tryptophane est choisi dans le groupe constitué de :
NT64L [L-néo-Trp11]NT(8-13),
SAR002D [D-Lys9,D-néo-Trp11,tert-Leu12]NT(9-13),
NT64D [D-néo-Trp11]NT(8-13),
NT73L [D-Lys9,L-néo-Trp11]NT(9-13),
NT65L [L-néo-Trp11, tert-Leu12]NT(8-13),
NT73D [D-Lys9,D-néo-Trp11]NT(9-13),
NT65D [D-néo-Trp11, tert-Leu12]NT(8-13),
NT74L [DAB9,L-néo-Trp11,tert-Leu12]NT(9-13),
NT66L [D-Lys8, L- néo-Trp11, tert-Leu12]NT(8-13),
NT74D [DAB9,Pro,D-néo-Trp11,tert-Leu12]NT(9-13),
NT66D [D-Lys8, D- néo-Trp11, tert-Leu12]NT(8-13),
NT75 L [DAB8,L-néo-Trp11]NT(8-13),
NT67L [D-Lys8, L-néo-Trp11]NT(8-13),
NT75D [DAB8,D-néo-Trp11]NT(8-13),
NT67D [D-Lys8, D-néo-Trp11]NT(8-13),
NT76L [D-Orn9,L-néo-Trp11]NT(8-13),
NT69L[N-méthyl-Arg8,L-Lys9,L-néo-Trp11,tert-Leu12]NT(8-13) ,
NT76D [D-Orn9,D-néo-Trp11]NT(8-13),
NT69D [N-méthyl-Arg8, L-Lys9,D-néo-Trp11,tert-Leu12]NT(8-13) ,
NT77L [D-Orn9,L-néo-Trp11,tert-Leu12]NT(8-13),
SAR001L [N- méthyl- Arg8,DAB9,L-néo-Trp11,tert-Leu12]NT(8-13) ,
NT77D [D-Orn9,D-néo-Trpll,tert-Leu12]NT(8-13),
SAR001D [N- méthyl- Arg8,DAB9,D-néo-Trp11,tert-leu12]NT(8-13),
NT78L [N-méthyl-Arg8,D-Orn9,L-néo-Trp11,tert-Leu12]NT(8-13) ,
SAR002L [D-Lys9,L-néo-Trp11,tert-Leu12]NT(9-13), et
NT78D [N-méthyl-Arg8,D-Orn9,D-néo-Trp11,tert-Leu12]NT(8-13).

21. Analgésique oculaire selon la revendication 14, comprenant en outre un opiacé.

22. Analgésique oculaire selon la revendication 21, dans lequel l'opiacé est choisi dans le groupe constitué de la morphine, la codéine, l'hydromorphone, l'oxymorphone, l'oxydodone, l'hydrocodone, la mépéridine, le lévorphanol, la méthadone, le sufentanil, l'alfentanil, le fentanyl, le rémifentanil, le lévométhadyl, la nalbuphine, la pentazocine, le butorphanol et la buprénorphine.

23. Analgésique oculaire selon la revendication 14, comprenant en outre une substance du groupe constitué des gouttes lubrifiantes pour les yeux, des larmes artificielles, des gouttes de méthylcellulose et des gouttes de morphine.

24. Analgésique oculaire selon la revendication 14, comprenant en outre une substance du groupe constitué d'un AINS, d'un stéroïde, d'un bêta bloquant, d'un agent antiviral, d'un immunosuppresseur et d'une cyclosporine.

25. Analgésique oculaire selon la revendication 14, dans lequel l'analgésique oculaire se présente sous la forme de gouttes ophtalmiques.
